# EUROPÄISCHE PATENTSCHRIFT

(11) **EP 2 298 196 B1**
(45) Veröffentlichungstag und Bekanntmachung des Hinweises auf die Patenterteilung: **06.07.2016**
(21) Anmeldenummer: 10009683.3
(22) Anmeldetag: 16.09.2010
(51) Int. Cl.: A61B 17/29

(54) **Medizinisches Instrument**
Medical instrument
Instrument médical

(30) Priorität: 21.09.2009 DE 102009042411
(43) Veröffentlichungstag der Anmeldung: 23.03.2011
(73) Patentinhaber: Richard Wolf GmbH, 75438 Knittlingen (DE)
(72) Erfinder: Prestel, Stephan, 76287 Rheinstetten-Mörsch (DE)
(74) Vertreter: Vollmann, Heiko

(56) Entgegenhaltungen:
- DE-U1- 9 320 558
- US-A1- 2002 156 497
- US-A1- 2004 073 210
- US-A1- 2004 193 212
- US-A1- 2004 249 411

## Beschreibung

Die Erfindung betrifft ein medizinisches Instrument mit den im Oberbegriff des Anspruchs 1 angegebenen Merkmalen.

Es sind Hohlschaftinstrumente, wie Manipulatoren, Zangen oder Scheren für den endoskopischen Einsatz bekannt, bei denen an dem distalen Ende eines Hohlschafts angeordnete bewegbare Instrumententeile über ein in dem Hohlschaft axial beweglich geführtes Betätigungselement mit einer an dem proximalen Ende des Hohlschafts angeordneten Handhabe bewegungsgekoppelt und so bedienbar sind. Um über einen Zugangskanal ins Körperinnere auch solche Behandlungsgebiete erreichen zu können, die seitlich einer durch den Zugangskanal definierten Zugangsachse liegen, sind Instrumente entwickelt worden, die distalseitig des Hohlschafts ein Endstück aufweisen, das relativ zu dem geraden Hohlschaft abwinkelbar ist. Allerdings erweist sich bei diesen Instrumenten bei abgewinkeltem Endstück die Kraftübertragung von der Handhabe zu dem bewegbaren Instrumententeil an dem abwinkelbaren Endstück als problematisch, da nur verhältnismäßig geringe Kräfte von einem Betätigungselement in dem Hohlschaft zu dem bewegbaren Instrumententeil übertragen werden können.

Zur Abwinklung des abwinkelbaren distalen Endstücks dieser Instrumente ist es bekannt, Steuerdrähte zu verwenden, die typischerweise distal beabstandet von einer Schwenkachse des Endstücks an diesem befestigt sind und zur Betätigung durch den Hohlschaft zu der proximalseitigen Handhabe geführt sind. Die Abwinkelbarkeit des Endstücks wird jedoch dadurch eingeschränkt, dass die sich beim Abwinkeln des Endstücks quer zu ihrer Längsachse verbiegenden Steuerdrähte nicht bis zum Erreichen einer Knickgrenze gebogen werden können, da das Endstück bei einem geknickten Steuerdraht nicht mehr zurück geschwenkt werden kann. Insofern können die Endstücke dieser Instrumente lediglich in einem vergleichsweise kleinen Winkelbereich abgewinkelt werden.

In DE 93 20 558 U1 ist ein endoskopisches Hohlschaftinstrument beschrieben. An dem distalen Ende des Hohlschafts dieses Instruments in ein Zangenmaul angeordnet und zwar an einem dort schwenkbar angelenkten Hebel. Dieser Hebel ist mit einer in dem Hohlschaft linear beweglich geführten Betätigungsstange bewegungsgekoppelt. Durch Verschieben der Betätigungsstange können der Hebel und das daran angelenkte Zangenmaul von einer Stellung, in der sie in direkter Verlängerung des Hohlschaftes ausgerichtet sind, in eine Stellung quer dazu verschwenkt werden. Zum Betätigen des Zangenmauls sind dessen beiden Maulteile jeweils als zweiteilige Hebel ausgebildet, wobei an dem proximalen Ende jedes der Maulteile ein Gelenkarm angelenkt ist. Die beiden Gelenkarme sind an ihren von den Maulteilen abgewandten Enden gelenkig mit einer Achse verbunden, die in ein Langloch eingreift, das an dem an dem Hohlschaft angelenkten Hebel ausgebildet ist. An der Achse ist eine Schubstange angelenkt, die über einen an dem Hohlschaft angelenkten zweiteiligen Hebel mit einer weiteren in dem Hohlschaft linear geführten Betätigungsstange bewegungsgekoppelt ist. Durch Verschieben dieser Betätigungsstange kann das Zangenmaul geöffnet und geschlossen werden.

Aus US 2004/0249411 A1 ist ein chirurgisches Instrument mit einem distalseitig eines Hohlschafts angeordneten Zangenmaul bekannt. Das Zangenmaul ist schwenkbar. Die beiden Maulteile des Zangenmauls sind gelenkig mit einander verbunden, derart, dass ein erstes der Maulteile einen zweiteiligen Hebel mit einem distalen und einem proximalen Maulteilabschnitt bildet, zwischen denen die gelenkige Verbindung mit dem zweiten Maulteil angeordnet ist. Das zweite Maulteil ist schwenkbar im Bereich seines proximalen Endes an dem distalen Ende des Hohlschafts angelenkt. Das zweite Maulteil ist über einen Gelenkarm mit einer in dem Hohlschaft linear beweglich geführten Betätigungsstange bewegungsgekoppelt. Durch Verschieben der Betätigungsstange kann das zweite Maulteil mit dem daran angelenkten ersten Maulteil, also das gesamte Zangenmaul von einer Stellung, in der das Zangenmaul in direkter Verlängerung des Hohlschafts angeordnet ist, in eine Stellung quer zum Hohlschaft verschwenkt werden. Um das Zangenmaul in beiden Stellungen öffnen und schließen zu können, ist das erste Maulteil über einen Gelenkarm, der an dem proximalen Ende des ersten Maulteils angelenkt ist, mit einer zweiten in dem Hohlschaft linear verschiebbar geführten Betätigungsstange bewegungsgekoppelt.

Aus US 2004/0193212 A1 ist ein chirurgisches Zangeninstrument gemäß dem Oberfegriff des Anspruchs 1 bekannt, bei dem an dem distalen Ende eines Hohlschafts ein abwinkelbar zu dem Hohlschaft angeordneter Instrumentenabschnitt angeordnet ist. Der abwinkelbare Instrumentenabschnitt ist mit einer in dem Hohlschaft linear beweglich geführten Betätigungsstange bewegungsgekoppelt und durch Verschieben der Betätigungsstange von einer Stellung, in der er in direkter Verlängerung des Hohlschafts angeordnet ist, in eine Stellung schräg dazu verschwenkbar. An dem abwinkelbaren Instrumentenabschnitt ist distalseitig ein erstes Maulteil eines Zangenmauls angelenkt, derart, dass das erste Maulteil einen zweiteiligen Hebel bildet. In dem ersten Maulteil ist das zweite Maulteil des Zangenmauls angelenkt. Dieses zweite Maulteil bildet ebenfalls einen zweiteiligen Hebel. Das zweite Maulteil ist an seinem proximalen Endbereich gelenkig mit einem Mehrgelenkarm verbunden, der wiederum mit einer zweiten in dem Hohlschaft linear beweglich geführten zweiten Betätigungsstange bewegungsgekoppelt ist. Durch Verschieben dieser zweiten Betätigungsstange können die beiden Maulteile zum Öffnen und Schließen des Zangenmauls verschwenkt werden.

US 2004/0073210 A1 ist ebenfalls ein chirurgisches Zangeninstrument zu entnehmen, bei dem an dem distalen Ende eines Hohlschafts ein abwinkelbar zu dem Hohlschaft angeordneter Instrumentenabschnitt angeordnet ist. Der abwinkelbare Instrumentenabschnitt ist mit eine in dem Hohlschaft linear beweglich geführten Betätigungsstange bewegungsgekoppelt und durch Verschieben der Betätigungsstange von einer Stellung, in der er in direkter Verlängerung des Hohlschafts angeordnet ist, in eine Stellung schräg dazu verschwenkbar. An dem distalen Ende des abwinkelbaren Instrumentenabschnitts sind die zwei Maulteile eines Zangenmauls angelenkt. Diese Maulteile sind über einen Mehrgelenkarm in einer zweiten in dem Hohlschaft verschiebbar geführten Betätigungsstange bewegungsgekoppelt, derart, dass das Zangenmaul durch Verschieben der Betätigungsstange in einer erste Richtung geöffnet und durch Verschieben der Betätigungsstange in eine zweite Richtung geschlossen werden kann.

In US 2002/0156497 A1 ist ein chirurgisches Instrument beschrieben, das ein Einführteil aufweist, an dessen distalen Ende ein erstes Maulteil eines Zangenmauls schwenkbar angelenkt ist. An dem ersten Maulteil ist ein zweites Maulteil des Zangenmauls angelenkt. Das erste Maulteil ist mit zwei an dem Einführteil in Längsrichtung verschiebbar geführten Betätigungsstangen gekoppelt, mit denen das Zangenmaul in eine zu dem Einführteil abgewinkelte Stellung geschwenkt werden kann. Zum Betätigen des Zangenmauls ist das zweite Maulteil des Zangenmauls über einen Mehrgelenkarm mit einer weiteren an dem Einführteil in Längsrichtung verschiebbar geführten Betätigungsstange bewegungsgekoppelt.

Die Aufgabe der Erfindung ist es, ein medizinisches Instrument der oben beschriebenen Art mit einem Hohlschaft und einem im Bereich des distalen Endes des Hohlschafts schwenkbar angeordneten Instrumentenabschnitts zu schaffen, das bei einem abgewinkelten Instrumentenabschnitt eine effiziente Kraftübertragung von einer an dem proximalen Ende des Hohlschafts angeordneten Handhabe zu dem an dem schwenkbaren Instrumentenabschnitt axial beweglich geführten Instrumententeil ermöglicht, wobei der schwenkbare Instrumentenabschnitt in Weiterbildung der Erfindung in einem größeren Winkelbereich als bislang bekannte Instrumente dieser Art abwinkelbar sein soll.

Gelöst wird diese Aufgabe durch ein medizinisches Instrument mit den im Anspruch 1 angegebenen Merkmalen. Vorteilhafte Weiterbildungen dieses Instruments ergeben sich aus den Unteransprüchen, der nachfolgenden Beschreibung sowie der Zeichnung. Hierbei können gemäß der Erfindung in den Unteransprüchen und der Beschreibung angegebene Merkmale jeweils für sich aber auch in Kombination die erfindungsgemäße Lösung gemäß Anspruch 1 weiter ausgestalten.

Das erfindungsgemäße medizinische Instrument weist einen Hohlschaft auf. In der Nähe des distalen Endes dieses Hohlschafts ist ein Instrumentenabschnitt abschwenkbar zu dem Hohlschaft angeordnet. In dem schwenkbaren Instrumentenabschnitt ist ein Instrumententeil axial beweglich gelagert. Dieses Instrumententeil, das ein Teil sein kann, mit dem direkt auf Organe, Gewebe und dergleichen eingewirkt werden kann oder vorzugsweise zur Bewegungskopplung mit einem solchen Teil in dem abwinkelbaren Instrumentenabschnitt dient, ist über einen zwangsgeführten Mehrgelenkarm mit einem in dem Hohlschaft axial beweglich geführten Betätigungsmittel bewegungsgekoppelt.

Der Mehrgelenkarm weist mindestens drei starre Armteile auf, die in Reihe gelenkig miteinander verbunden sind. In diesem Sinne ist unter einem Mehrgelenkarm auch eine Gelenkkette zu verstehen, wie sie z. B. in Kettentrieben verwendet wird. Mit dem Gelenkarm stellt die Erfindung ein Verbindungselement zwischen dem in dem Hohlschaft angeordneten Betätigungsmittel und dem in dem schwenkbaren Instrumentenabschnitt angeordneten beweglich gelagerten Instrumententeil zur Verfügung, das an eine Abwickelung des schwenkbaren Instrumentenabschnitts angepasst abwinkelbar ist.

Aufgrund der Zwangsführung ist die Abwinklung der einzelnen Armteile des Mehrgelenkarms beim Verschwenken des schwenkbaren Instrumentenabschnitts vorgegeben und kann im Zusammenwirken mit der Wahl einer geeigneten Anzahl von Armteilen und geeigneter Armteilabmessungen vorteilhaft so gewählt werden, dass der Mehrgelenkarm während des Abwinkelns und nach dem Abwinkeln zumindest weitestgehend innerhalb des Innenquerschnitts von Hohlschaft und schwenkbarem Instrumentenabschnitt angeordnet ist und eine besonders effiziente Kraftübertragung von dem in dem Hohlschaft geführten Betätigungsmittel zu dem beweglich in dem schwenkbaren Instrumentenabschnitt gelagerten Instrumententeil ermöglicht. Vorteilhaft kann der Mehrgelenkarm auch im abgewinkelten Zustand gleichermaßen Druckund Zugkräfte von dem Betätigungsmittel auf das in dem schwenkbaren Instrumentenabschnitt beweglich gelagerte Instrumententeil übertragen.

Das in Achsrichtung des Hohlschafts in diesem geführte Betätigungsmittel kann zweckmäßigerweise derart ausgebildet sein, dass es von einer proximalseitig des Hohlschafts angeordneten Handhabe bedient werden kann.

Der Mehrgelenkarm weist mindestens drei in Reihe miteinander gelenkig verbundene Armteile auf, wobei mindestens ein zwischen zwei Armteilen angeordnetes Armteil um eine Achse schwenkbar ist, die an dem Hohlschaft beabstandet von einer geraden Verbindungslinie der Anlenkpunkte benachbarter Armteile an diesem mittleren Armteil angeordnet ist. So ist an einem ersten Armteil, das an dem in dem Hohlschaft geführten Betätigungsmittel angelenkt ist, beabstandet von der Anlenkung an dem Betätigungsmittel ein zweites Armteil angelenkt sein, das wiederum beabstandet von der gelenkigen Verbindung mit dem ersten Armteil mit einem dritten Armteil gelenkig verbunden ist, wobei an dem dritten Armteil in Abstand zu der gelenkigen Verbindung mit dem zweiten Armteil das in dem schwenkbaren Instrumentenabschnitt beweglich gelagerte Instrumententeil angelenkt ist. Bei dieser Ausgestaltung bildet das zweite Armteil einen normal zur Verbindungslinie dessen Anlenkpunkte mit dem ersten und dem dritten Armteil ausgerichteten Hebelarm, der im Bereich seines von der Verbindungslinie der Anlenkpunkte mit dem benachbarten Armteilen abgewandten Endes schwenkbar an dem Hohlschaft angelenkt ist. Insofern ist der mögliche Bewegungsweg des zweiten Armteils vorgegeben und der aus dem ersten, zweiten und dritten Armteil bestehende Mehrgelenkarm zwangsgeführt.

Eine besonders effektive Kraftübertragung von dem in dem Hohlschaft geführten Betätigungsmittel zu dem in dem schwenkbaren Instrumentenabschnitt beweglich gelagerten Instrumententeil und eine möglichst eng an die Anwinklung des schwenkbaren Instrumentenabschnitts angeglichene Abwinklung des Mehrgelenkteils kann dann erzielt werden, wenn, wie es weiter vorteilhaft vorgesehen ist, zusätzlich zu der Zwangsführung an dem Hohlschaft mindestens ein weiteres zwischen zwei Armteilen angeordnetes Armteil um eine Achse schwenkbar ist, die vorteilhafterweise aber an dem schwenkbaren Instrumentenabschnitt beabstandet von einer geraden Verbindungslinie der Anlenkpunkte benachbarter Armteile dieses Armteils angeordnet ist. Hierbei bildet das an dem schwenkbaren Instrumentenabschnitt angelenkte Armteil eine weitere Zwangsführung des Mehrgelenkarms an dem schwenkbaren Instrumentenabschnitt. Demzufolge ist der Mehrgelenkarm bei dieser Ausgestaltung sowohl an dem Hohlschaft als auch an dem schwenkbaren Instrumentenabschnitt zwangsgeführt. Zweckmäßigerweise weist der Mehrgelenkarm hierbei zumindest fünf Armteile auf, wobei das die Zwangsführung an dem schwenkbaren Instrumentenabschnitt bildende Armteil typischerweise nicht direkt mit dem die Zwangsführung an dem Hohlschaft bildenden Armteil verbunden ist.

Zum Abwinkeln des schwenkbaren Instrumentenabschnitts relativ zu dem Hohlschaft ist bevorzugt ein Hebel vorgesehen, der an einem distalen Endbereich eines zweiten in dem Hohlschaft axial beweglich geführten Betätigungsmittel und an dem schwenkbaren Instrumentenabschnitt angelenkt ist. D. h., das zweite axial bewegliche Betätigungsmittel bildet mit dem Hebel und dem schwenkbaren Instrumentenabschnitt eine schubkurbelartige Mechanik, mit der die Linearbewegung des in dem Hohlschaft angeordneten Betätigungsmittels in eine Schwenkbewegung des schwenkbaren Instrumentenabschnitts umgewandelt wird. Mittels dieser Mechanik kann der schwenkbare Instrumentenabschnitt in einem deutlich größeren Winkelbereich abgewinkelt werden, als dies bei den bislang bekannten Instrumenten der Fall ist, die zum Abwinkeln eines distalen Instrumentabschnitts Steuerdrähte verwenden. Um das Verschwenken des an dem Betätigungsmittel angelenkten Hebels zu ermöglichen, kann es zweckmäßig sein, den Hohlschaft an seinem distalen Ende außenseitig des Hebels mit einem Längsschlitz zu versehen.

Zweckmäßigerweise kann der Hohlschaft so ausgestaltet sein, dass sein distales Ende eine Gabel bildet, an der der abwinkelbare Instrumentenabschnitt angelenkt ist. Dann können an dem distalen Ende eines den Hohlschaft bildenden starren Rohres zwei sich in Längsrichtung des Rohres erstreckende Fortsätze ausgebildet sein, die einander an dem Rohr direkt gegenüber liegen. Zwischen diesen Fortsätzen kann der schwenkbare Instrumentenabschnitt schwenkbar gelagert sein, wobei die Anlenkung des schwenkbaren Instrumentenabschnitts bevorzugt an zwei Stiften erfolgt, die an den einander zugewandten Innenseiten der Fortsätze des Hohlschafts derart in Richtung einer Mittelachse des Hohlschafts ragen, dass sie eine gemeinsame Schwenkachse für den schwenkbaren Instrumentenabschnitt bilden. Die an dem Hohlschaft ausgebildeten Fortsätze sind günstigerweise so dimensioniert, dass sie ein Verschwenken des schwenkbaren Instrumentenabschnitts in einem Winkelbereich von etwa 90° quer zur Mittelachse des Hohlschafts ermöglichen, sodass mit dem erfindungsgemäßen Instrument Organ- und Gewebemanipulationen auch seitlich und sogar quer zum Zugangskanal möglich sind, über den das Instrument in das Körperinnere geführt wird.

Bevorzugt bildet ein Rohrteil eine äußere Hülle des schwenkbaren Instrumentenabschnitts. In diesem Rohrteil kann das mit dem Mehrgelenkarm bewegungsgekoppelte Instrumententeil des schwenkbaren Instrumentenabschnitts beweglich gelagert sein. Ferner kann dieses Rohrteil zur Aufnahme eines Teils des Mehrgelenkarms dienen. Um beim Abwinkeln des Mehrgelenkarms dessen in dem Rohrteil angeordnete Armteile auch über die Innenkontur des Rohrteils hinaus auslenken zu können, kann das Rohrteil in einem dem Mehrgelenkarm direkt gegenüberliegenden, d. h. direkt außenseitig des Mehrgelenkarms angeordneten Bereich längsgeschlitzt ausgebildet sein.

Bei dem erfindungsgemäßen medizinischen Instrument handelt es sich vorzugsweise um ein solches Instrument, das ein distales Instrumentenmaul mit zwei relativ zueinander bewegbaren Branchen aufweist. So kann das erfindungsgemäße Instrument eine Fasszange oder eine Schere sein, bei denen zumindest eine Branche mit dem beweglich im schwenkbaren Instrumentenabschnitt gelagerten Instrumententeil bewegungsgekoppelt ist. Des Weiteren kann das erfindungsgemäße Instrument bevorzugt auch ein Organmanipulator mit einem distalseitig angeordneten fächerförmig aufspreizbaren Spreizkörper sein, bei dem einzelne oder mehrere Spreizarme des Spreizkörpers mit dem beweglich im schwenkbaren Instrumentenabschnitt gelagerten Instrumententeil bewegungsgekoppelt sind.

Nachfolgend ist die Erfindung anhand von in der Zeichnung dargestellten Ausführungsbeispielen näher erläutert. In der Zeichnung zeigt:
- Fig. 1: schematisch, in perspektivischer Ansicht eine medizinische Fasszange,
- Fig. 2: schematisch, in perspektivischer Ansicht eine medizinische Schere,
- Fig. 3: in einer Unteransicht einen distalen Endbereich eines medizinischen Instruments mit einem schwenkbaren Instrumentenabschnitt in einer ersten Arbeitsstellung,
- Fig. 4: den distalen Endbereich nach Fig. 3 in einer Seitenansicht,
- Fig. 5: eine Schnittansicht entlang der Linie V-V in Fig. 3,
- Fig. 6: in einer Unteransicht den distalen Endbereich des medizinischen Instruments nach Fig. 3 in einer zweiten Arbeitsstellung,
- Fig. 7: den distalen Endbereich des medizinischen Instruments nach Fig. 6 in einer Seitenansicht,
- Fig. 8: eine Schnittansicht entlang der Linie VIII-VIII in Fig. 6,
- Fig. 9: in einer Unteransicht den distalen Endbereich des medizinischen Instruments nach Fig. 3 in einer dritten Arbeitsstellung,
- Fig. 10: den distalen Endbereich des medizinischen Instruments nach Fig. 9 in einer Seitenansicht,
- Fig. 11: eine Schnittansicht entlang der Linie XI-XI in Fig. 9,
- Fig. 12: den distalen Endbereich des medizinischen Instruments nach Fig. 9 in einer Frontansicht,
- Fig. 13: in einer Unteransicht den distalen Endbereich des medizinischen Instruments nach Fig. 3 in einer vierten Arbeitsstellung,
- Fig. 14: den distalen Endbereich des medizinischen Instruments nach Fig. 13 in einer Seitenansicht,
- Fig. 15: eine Schnittansicht entlang der Linie XV-XV in Fig. 13, und
- Fig. 16: den distalen Endbereich des medizinischen Instruments nach Fig. 13 in einer Frontansicht.

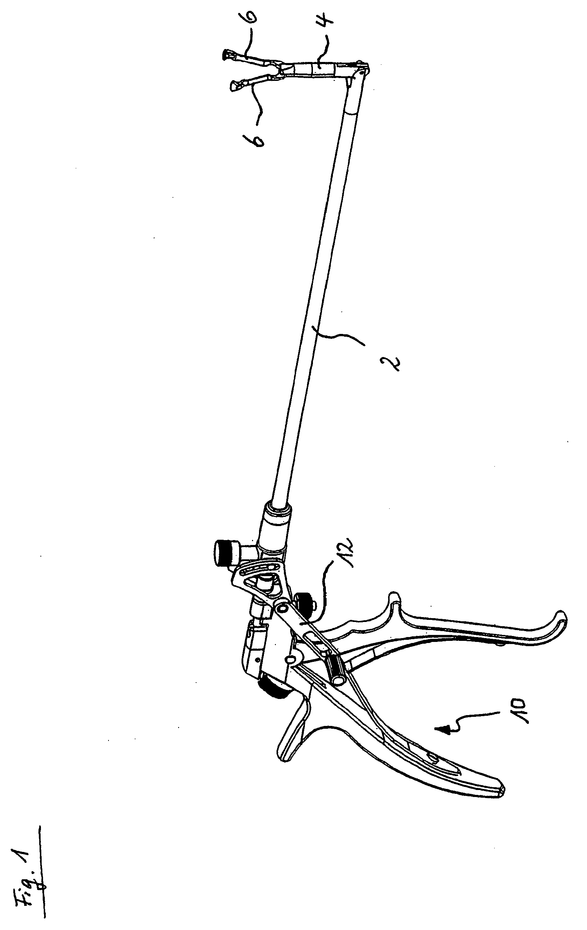

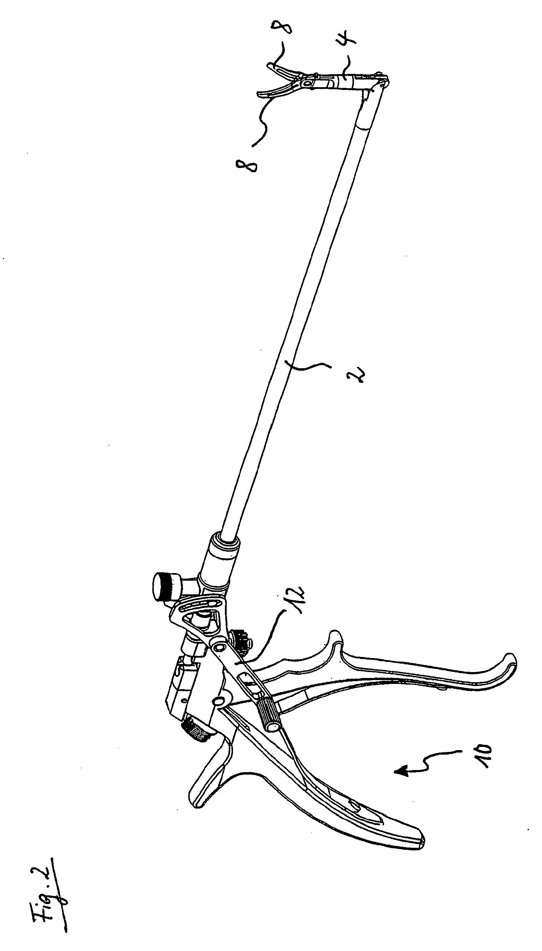

Sowohl die in Fig. 1 dargestellte medizinische Fasszange als auch die in Fig. 2 dargestellte medizinische Schere weisen jeweils einen Hohlschaft 2 auf, der von einem starren, geraden Rohr gebildet wird. In der Nähe des distalen Endes des Hohlschafts 2 ist an diesem jeweils ein Instrumentenabschnitt 4 in einem Winkelbereich von im Wesentlichen bis zu 90 ° quer zu einer Längsachse des Hohlschafts 4 abwinkelbar angelenkt. Im Bereich des distalen Endes des Instrumentenabschnitts 4 sind bei der in Fig. 1 dargestellten Fasszange Branchen 6 und bei der in Fig. 2 dargestellten Schere Schneiden 8 schwenkbeweglich gelagert. Mittels in dem Instrumentenabschnitt 4 und dem Hohlschaft 2 angeordneter Betätigungsmittel, auf die nachfolgend detaillierter eingegangen wird, sind die Branchen 6 bzw. die Schneiden 8 mit einer Handhabe 10, die proximalseitig des Hohlschafts 2 angeordnet ist, bewegungsgekoppelt. Darüber hinaus sind an dem Instrumentenabschnitt 4 und in dem Hohlschaft 2 weitere Betätigungsmittel angeordnet, die weiter unten noch im Einzelnen beschrieben sind und mit denen der Instrumentenabschnitt 4 relativ zu dem Hohlschaft 2 abgewinkelt werden kann. Diese Betätigungsmittel sind mit einem Betätigungshebel 12 bewegungsgekoppelt, der proximalseitig des Hohlschafts 2 im Bereich der Handhabe 10 angeordnet ist.

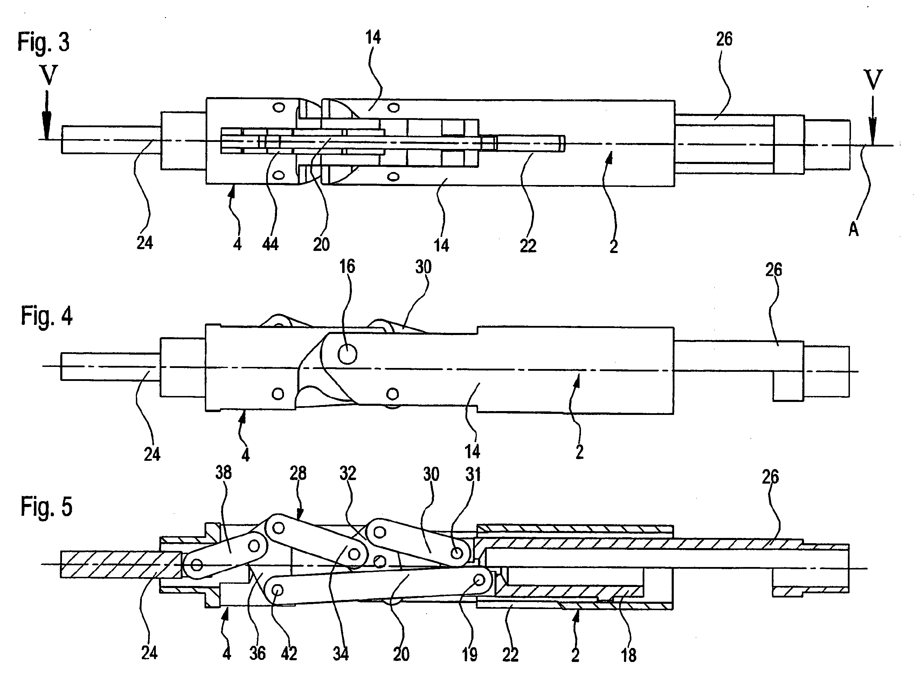

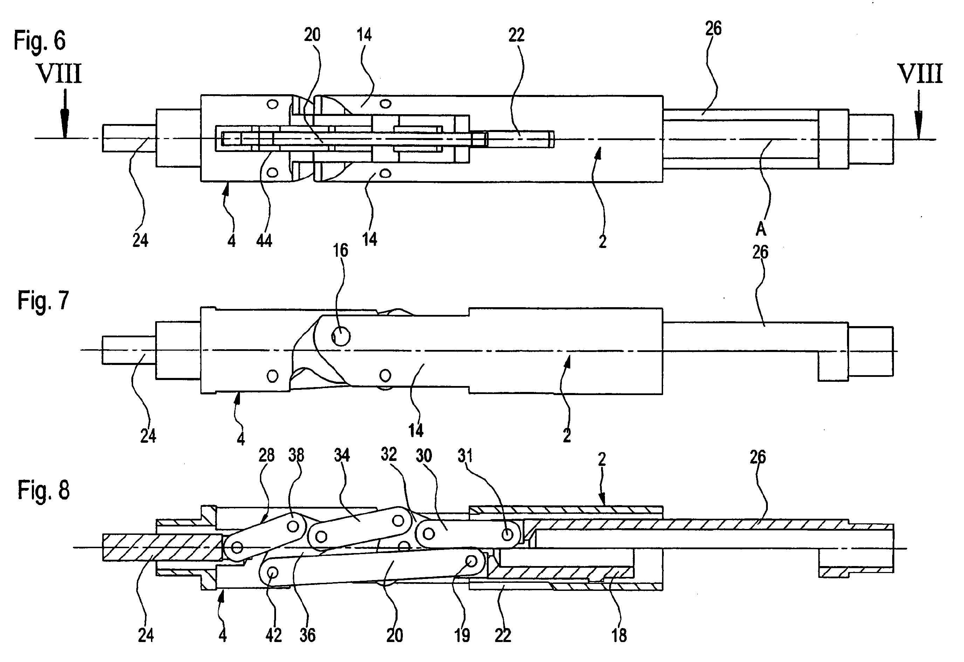

Die Figuren 3 bis 16 zeigen vergrößert die Anbindung des Instrumentenabschnitts 4 an dem Hohlschaft 2 der Fasszange nach Fig. 1 bzw. der Schere nach Fig. 2. An dem distalen Ende des Hohlschafts bildet dieser eine Gabel mit zwei Fortsätzen 14. Diese Fortsätze 14 sind in Längsrichtung des Hohlschafts 2 ausgerichtet und einander diametral gegenüberliegend angeordnet. An jedem der Fortsätze 14 ist jeweils eine Bohrung 16 ausgebildet, wobei die Bohrungen 16 eine gemeinsame Mittelachse aufweisen. Die Bohrungen 16 dienen zur Aufnahme von Gelenkstiften, mit denen der abwinkelbare Instrumentenabschnitt 4 an einem proximalen Endbereich, der zwischen den Fortsätzen 14 angeordnet ist, schwenkbar mit dem Hohlschaft 2 verbunden ist.

Zum Verschwenken des Instrumentenabschnitts 4 ist in dem Hohlschaft 2 ein längliches Betätigungsmittel 18 in Form eines in dem Hohlschaft 2 axial verschiebbar geführten Schiebers 18 angeordnet, der im Bereich seines proximalen Endes mit dem Betätigungshebel 12 bewegungsgekoppelt ist. Nahe dem distalen Ende des Schiebers 18 ist an diesem über einen Gelenkstift 19 ein Hebel 20 angelenkt. Im Bereich seines von der Anlenkung an dem Schieber 18 abgewandten Endes ist der Hebel 20 über einen Gelenkstift 42 an dem schwenkbaren Instrumentenabschnitt 4 angelenkt. Um eine ausreichende Schwenkbewegung des Hebels 20 quer zu einer Mittelachse A des Hohlschafts 2 zu ermöglichen, ist an dem Hohlschaft 2 an einer dem Schieber 18 und dem Hebel 20 direkt gegenüberliegenden Unterseite proximalseitig an dem zwischen den Fortsätzen 14 ausgebildeten Zwischenraum ein Schlitz 22 ausgebildet, der sich parallel zu der Mittelachse A des Hohlschafts 2 erstreckt.

In dem schwenkbaren Instrumentenabschnitt 4 ist ein Instrumententeil 24 in axialer Richtung des Instrumentenabschnitts 4 linear bewegbar gelagert. Bei dem Instrumententeil 24 handelt es sich um eine Zug-Druck-Stange, die mit einer nicht dargestellten, zum Stand der Technik zählenden Mechanik zum Verschwenken der Branchen 6 der in Fig. 1 dargestellten Fasszange bzw. zum Verschwenken der Schneiden 8 der in Fig. 2 dargestellten Schere bewegungsgekoppelt ist.

Zum Bewegen des Instrumententeils 24 ist in dem Hohlschaft 2 ein weiteres längliches Betätigungsmittel 26 angeordnet, das von einem in dem Hohlschaft 2 axial beweglich geführten Schieber 26 gebildet wird. Mit diesem Schieber 26 ist das Instrumententeil 24 in dem Instrumentenabschnitt 4 über einen Mehrgelenkarm 28 gelenkig verbunden. Ein erstes längliches Armteil 30 des Mehrgelenkarms 28 ist an dem Schieber 26 im Bereich dessen distalen Endes über einen Gelenkstift 31 angelenkt. Im Bereich des von der Anlenkung an dem Schieber 26 abgewandten Endes des Armteils 30 sind an diesem zwei Armteile 32 angelenkt, wobei die Armteile 32 an von einander abgewandten Flachseiten des Armteils 30 befestigt sind. An den von der Anlenkung an dem Armteil 30 abgewandten Enden der Armteile 32 ist zwischen den Armteilen 32 ein längliches Armteil 34 angelenkt, wobei im Bereich eines von dieser Anlenkung abgewandten Endes des Armteiles 34 zwei Armteile 36 an von einander abgewandten Flachseiten des Armteils 34 befestigt sind. Im Bereich der von der Anlenkung an dem Armteil 34 der Armteile 36 abgewandten Enden der Armteile 36 ist zwischen diesen schließlich ein weiteres Armteil 38 angelenkt, das wiederum mit dem Instrumententeil 24 schwenkbeweglich verbunden ist.

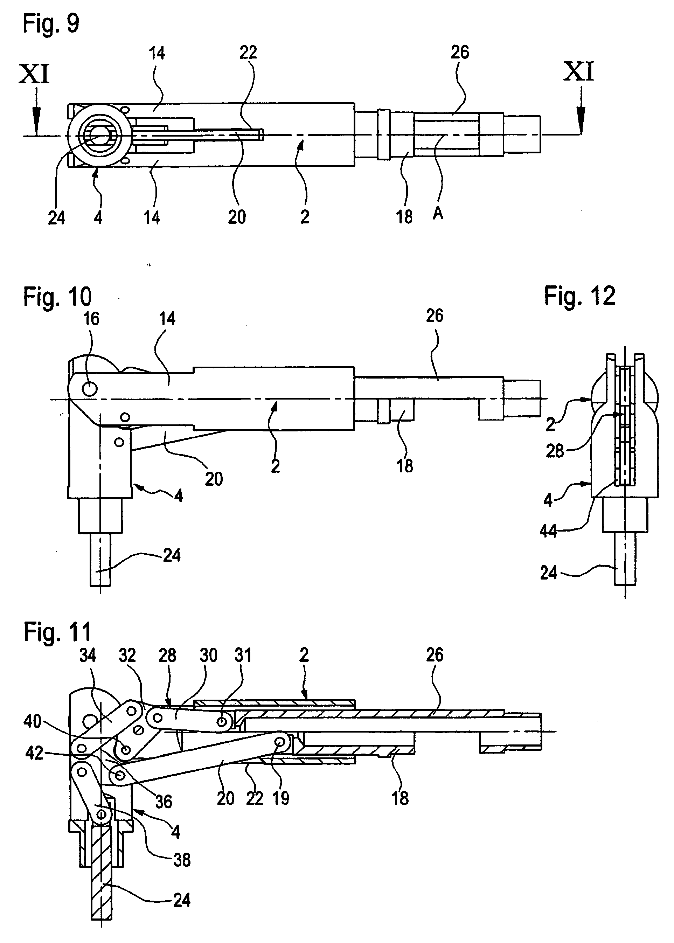

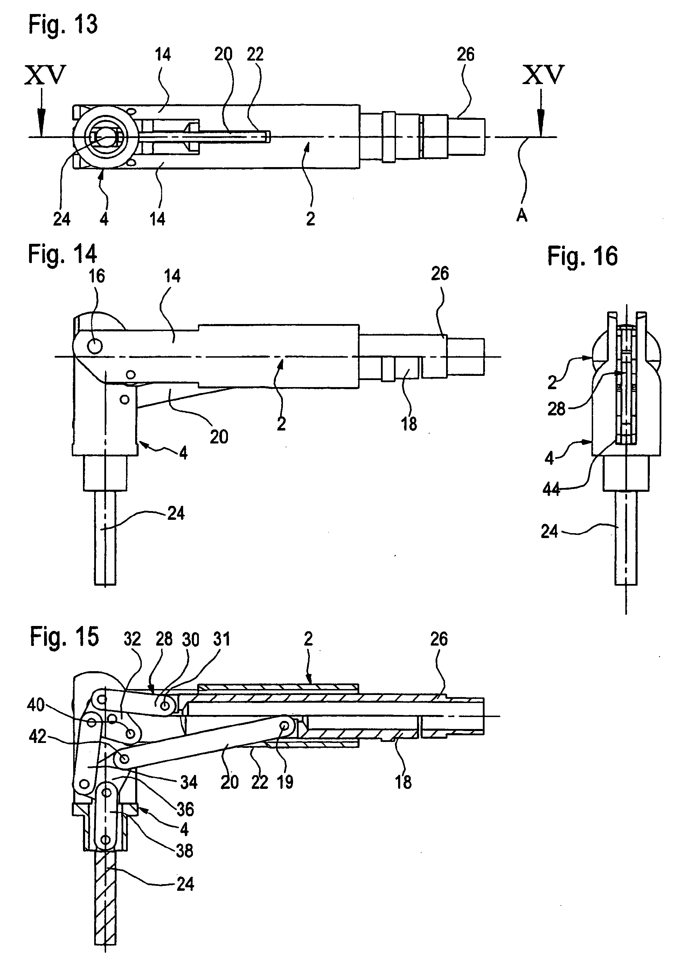

Die beiden Armteile 32 sowie die beiden Armteile 36 werden von abgerundet und in Draufsicht (Fig. 11, 15) im Wesentlichen dreieckigen Platten gebildet, wobei an dem Armteil 32 eine Verbindungslinie der Anlenkpunkte mit den Armteilen 30 und 34 im Wesentlichen parallel zu einer Seitenkante des Armteils 32 angeordnet ist und an dem Armteil 36 eine Verbindungslinie der Anlenkpunkte mit den Armteilen 34 und 38 im Wesentlichen parallel zu einer Seitenkante des Armteils 36 angeordnet ist. Im Bereich einer im Wesentlichen senkrecht von der Verbindungslinie von Anlenkpunkten mit benachbarten Armteilen beabstandet angeordneten Ecke sind die Armteile 32 über einen Gelenkstift 40 (Fig. 11 und 16) an dem Hohlschaft 2 und die Armteile 36 über einen Gelenkstift 42 an dem Instrumentenabschnitt 4 schwenkbar befestigt. Der Gelenkstift 42 dient zudem zur Anlenkung des Hebels 20 an dem Instrumentenabschnitt 4. Um bei einem Abwinkeln des Mehrgelenkarms 28 die Armteile 30, 32, 34 und 36 auch über die Innenkontur des Instrumentenabschnitts 4 auslenken zu können, weist der Instrumentenabschnitt 4 direkt außenseitig des Mehrgelenkarms 28 einen Längsschlitz 44 auf.

Die Funktionsweise der in den Figuren 3 bis 16 dargestellten Mechanik ist wie folgt:
Um den Instrumentenabschnitt 4 von der in den Figuren 3 bis 8 dargestellten Stellung, in der der Instrumentenabschnitt 4 eine direkte gerade Verlängerung des Hohlschafts 2 bildet, in die in den Figuren 9 bis 16 dargestellte und um etwa 90° abgewinkelte Stellung zu verschwenken, wird der Schieber 18 mittels des Betätigungshebels 12 in proximaler Richtung verschoben, wobei der Instrumentenabschnitt 4 durch Auslenken des Hebels 20 in einem Winkel von ungefähr 90° verschwenkt wird. Gleichzeitig mit dem Verschwenken des Instrumentenabschnitts 4 werden auch die Armteile 32 um eine von dem Gelenkstift 40 gebildete Schwenkachse und die Armteile 36 über eine von dem Gelenkstift 42 gebildete Schwenkachse verschwenkt, wodurch der Mehrgelenkarm 28 korrespondierend zur Abwinklung des Instrumentenabschnitts 4 seine Form ändert, ohne dass das in dem Instrumentenabschnitt 4 beweglich gelagerte Instrumententeil 24 und der Schieber 18 wesentlich bewegt werden. Das Verschwenken des Instrumentenabschnitts 4 von der in den Figuren 9 bis 16 dargestellten abgewinkelten Stellung in die nicht abgewinkelte Stellung erfolgt in umgekehrter Weise, indem der Schieber 18 mittels des Betätigungshebels 12 in distaler Richtung bewegt wird, wodurch der Instrumentenabschnitt 4 von dem an dem Schieber 18 angelenkten Hebel 20 in eine Stellung verschwenkt wird, in der er in gerader Verlängerung des Hohlschafts 2 angeordnet ist.

Das in dem Instrumentenabschnitt 4 beweglich gelagerte Instrumententeil 24, das mit den Branchen 6 der in Figur 1 dargestellten Fasszange bzw. mit den Schneiden 8 der in Figur 2 dargestellten Schere zum Öffnen und Schließen des Zangen- bzw. Scherenmauls bewegungsgekoppelt ist, kann in distaler Richtung in dem Instrumentenabschnitt 4 verschoben werden, indem der Schieber 26 durch Betätigen der Handhabe 10 in distaler Richtung verschoben wird, wobei die Bewegung des Schiebers 26 von dem Mehrgelenkarm 28 auf das Instrumententeil 24 übertragen wird. Umgekehrt kann das Instrumententeil 24 in proximaler Richtung verschoben werden, indem der Schieber 26 durch Betätigen der Handhabe 10 in proximaler Richtung verschoben wird. Die Ausgestaltung des Mehrgelenkarms 28 erlaubt diese Bewegungen in distaler und proximaler Richtung sowohl dann, wenn der Instrumentenabschnitt 4 in direkter gerader Verlängerung des Hohlschafts 2 angeordnet ist als auch dann, wenn der Instrumentenabschnitt 4 gegenüber dem Hohlschaft 2 abgewinkelt ist sowie in jeder Zwischenstellung.

### Bezugszeichenliste

- 2: - Hohlschaft
- 4: - Abschnitt
- 6: - Branche
- 8: - Schneide
- 10: - Handhabe
- 12: - Betätigungshebel
- 14: - Fortsatz
- 16: - Bohrung
- 18: - Betätigungsmittel, Schieber
- 19: - Gelenkstift
- 20: - Hebel
- 22: - Schlitz
- 24: - Instrumententeil
- 26: - Betätigungsmittel, Schieber
- 28: - Mehrgelenkarm
- 30: - Armteil
- 31: - Gelenkstift
- 32: - Armteil
- 34: - Armteil
- 36: - Armteil
- 38: - Armteil
- 40: - Gelenkstift
- 42: - Gelenkstift
- 44: - Längsschlitz
- A: - Mittelachse

## Patentansprüche

1. Medizinisches Instrument mit einem Hohlschaft (2) und einem nahe dem distalen Ende des Hohlschafts (2) abwinkelbar zu dem Hohlschaft (2) angeordneten Instrumentenabschnitt (4) mit einem darin axial beweglich gelagerten Instrumententeil (24), **dadurch gekennzeichnet, dass** das Instrumententeil (24) über einen zwangsgelenkten Mehrgelenkarm (28) mit einem in dem Hohlschaft (2) axial beweglich geführten Betätigungsmittel (26) bewegungsgekoppelt ist, wobei der Mehrgelenkarm (28) mindestens drei in Reihe miteinander gelenkig verbundene Armteile (30, 32, 34, 36, 38) aufweist, wobei mindestens ein zwischen zwei Armteilen (30, 34) angeordnetes Armteil (32) um eine Achse schwenkbar ist, die an dem Hohlschaft (2) beabstandet von einer geraden Verbindungslinie der Anlenkpunkte benachbarter Armteile(30, 34) an dem zwischen den zwei Armteilen (30, 34) angeordneten Armteil (32) angeordnet ist.

2. Medizinisches Instrument nach Anspruch 1, **dadurch gekennzeichnet, dass** mindestens ein weiteres zwischen zwei Armteilen (34, 38) angeordnetes Armteil (36) um eine Achse schwenkbar ist, die an dem schwenkbaren Instrumentenabschnitt (4) beabstandet von einer geraden Verbindungslinie der Anlenkpunkte benachbarter Armteile (34, 38) dieses Armteils (36) angeordnet ist.

3. Medizinisches Instrument nach einem der vorangehenden Ansprüche, **dadurch gekennzeichnet, dass** in dem Hohlschaft (2) ein zweites Betätigungsmittel (18) axial beweglich geführt ist, wobei ein Hebel (20) an einem distalen Endbereich des Betätigungsmittels (18) und an dem abwinkelbaren Instrumentenabschnitt (4) angelenkt ist.

4. Medizinisches Instrument nach einem der vorangehenden Ansprüche, **dadurch gekennzeichnet, dass** das distale Ende des Hohlschafts (2) eine Gabel bildet, an der der abwinkelbare Instrumentenabschnitt (4) angelenkt ist.

5. Medizinisches Instrument nach einem der vorangehenden Ansprüche, **dadurch gekennzeichnet, dass** ein Rohrteil eine äußere Hülle des schwenkbaren Instrumentenabschnitts (4) bildet.

6. Medizinisches Instrument nach Anspruch 5, **dadurch gekennzeichnet, dass** das Rohrteil in einem dem Mehrgelenkarm (24) direkt gegenüberliegenden Bereich längstgeschlitzt ausgebildet ist.

7. Medizinisches Instrument nach einem der vorangehenden Ansprüche, **dadurch gekennzeichnet, dass** das Instrument eine Fasszange ist.

8. Medizinisches Instrument nach einem der Ansprüche 1 bis 6, **dadurch gekennzeichnet, dass** das Instrument eine Schere ist.

9. Medizinisches Instrument nach einem der Ansprüche 1 bis 6, **dadurch gekennzeichnet, dass** das Instrument eine Organmanipulator ist.

## Claims

1. A medical instrument with a hollow shank (2) and with an instrument section (4) with an instrument part (24) which is axially movably mounted in said instrument section (4), said instrument section, close to the distal end of the hollow shank (2), being arranged in a bendable manner to the hollow shank (2), **characterised in that** the instrument part (24) is coupled in movement to an actuation means (26) which is axially movably guided in the hollow shank (2), via a forcibly guided multi-joint arm (28), wherein the multi-joint arm (28) comprises at least three arm parts (30, 32, 34, 36, 38) which are connected in series to one another in an articulated manner, wherein at least one arm part (32), which is arranged between two arm parts (30, 34), is pivotable about an axis which is arranged on the hollow shank (2), distanced to a straight connection line of the articulation points of adjacent arm parts (30, 34) to the arm part (32) arranged between the two arm parts (30, 34).

2. A medical instrument according to claim 1, **characterised in that** at least one further arm part (36) arranged between two arm parts (34, 38), is pivotable about an axis which is arranged on the pivotable instrument section (4), distanced to a straight connection line of the articulation points of adjacent arm parts (34, 38) to this arm part (36).

3. A medical instrument according to one of the preceding claims, **characterised in that** a second actuation means (18) is axially movably guided in the hollow shank (2), wherein a lever (20) is articulated on a distal end region of the actuation means (18) and on the bendable instrument shank (4).

4. A medical instrument according to one of the preceding claims, **characterised in that** the distal end of the hollow shank (2) forms a fork, on which the bendable instrument section (4) is articulated.

5. A medical instrument according to one of the preceding claims, **characterised in that** a tube part forms an outer envelope of the pivotable instrument section (4).

6. A medical instrument according to claim 5, **characterised in that** the tube part is designed in a longitudinally slotted manner in a region lying directly opposite the multi-joint arm (24).

7. A medical instrument according to one of the preceding claims, **characterised in that** the instrument is a gripper forceps.

8. A medical instrument according to one of the claims 1 to 6, **characterised in that** the instrument is a scissors.

9. A medical instrument according to one of the claims 1 to 6, **characterised in that** the instrument is an organ manipulator.

## Revendications

1. Instrument médical avec une tige creuse (2) et une partie pour instrument (4) disposée de manière angulairement mobile proche de l'extrémité distale de la tige creuse (2) et comportant une partie d'instrument (24) montée là-dedans de manière axialement mobile, **caractérisé en ce que** la partie d'instrumaent (24) est accouplée en mouvement, via un bras (28) à articulations multiples et à articulation forcée, à un organe d'actionnement (26) guidé de manière axialement mobile dans la tige creuse (2), le bras à articulations multiples (28) comprenant au moins trois parties de bras (30, 32, 34, 36, 38) reliées les unes aux autres en série de manière articulée, au moins une partie de bras (32) disposée entre deux parties de bras (30, 34) étant pivotante autour d'un axe qui est disposé sur la tige creuse (2), de manière espacée d'une ligne de liaison droite des points d'articulation de parties de bras avoisinantes (30, 34), sur la partie de bras (32) disposée entre les deux parties de bras (30, 34).

2. Instrument médical selon la revendication 1, **caractérisé en ce qu'**au moins une autre partie de bras (36) disposée entre deux parties de bras (34, 38) est pivotante autour d'un axe qui est disposé sur la partie pivotante (4) pour instrument, de manière espacée d'une ligne de liaison droite des points d'articulation de parties de bras avoisinantes (34, 38) à cette partie de bras (36).

3. Instrument médical selon l'une des revendications précédentes, **caractérisé en ce que**, dans la tige creuse (2), un second organe d'actionnement (18) est guidé de manière axialement mobile, un levier (20) étant articulé dans une zone d'extrémité distale de l'organe d'actionnement (18) et sur la partie pour instrument (4) angulairement mobile.

4. Instrument médical selon l'une des revendications précédentes, **caractérisé en ce que** l'extrémité distale de la tige creuse (2) forme une fourchette sur laquelle la partie pour instrument (4) angulairement mobile est articulée.

5. Instrument médical selon l'une des revendications précédentes, **caractérisé en ce qu'**une pièce tubulaire forme une gaine extérieure de la partie pour instrument (4) pivotante.

6. Instrument médical selon la revendication 5, **caractérisé en ce que** la pièce tubulaire est formée avec une fente longitudinale dans une zone directement en regard du bras à articulations multiples (24).

7. Instrument médical selon l'une des revendications précédentes, **caractérisé en ce que** l'instrument est une pince de préhension.

8. Instrument médical selon l'une des revendications 1 à 6, **caractérisé en ce que** l'instrument est constitué par des ciseaux.

9. Instrument médical selon l'une des revendications 1 à 6, **caractérisé en ce que** l'instrument est un manipulateur d'organe.
